(19) Europäisches Patentamt
European Patent Office
Office européen des brevets

(11) EP 1 287 057 B1

(12) **EUROPÄISCHE PATENTSCHRIFT**

(45) Veröffentlichungstag und Bekanntmachung des Hinweises auf die Patenterteilung:
**18.02.2004 Patentblatt 2004/08**

(21) Anmeldenummer: **01936409.0**

(22) Anmeldetag: **30.05.2001**

(51) Int Cl.7: **C08G 77/388**, C08L 83/08, A61K 6/10

(86) Internationale Anmeldenummer:
**PCT/EP2001/006140**

(87) Internationale Veröffentlichungsnummer:
**WO 2001/092373 (06.12.2001 Gazette 2001/49)**

# (54) AZIRIDINOSILIKONE UND DEREN VERWENDUNG

AZIRIDINOSILICONES AND THE USE THEREOF

AZIRIDINOSILICONES ET LEUR UTILISATION

(84) Benannte Vertragsstaaten:
**AT BE CH CY DE DK ES FI FR GB GR IE IT LI LU MC NL PT SE TR**

(30) Priorität: **31.05.2000 DE 10026857**

(43) Veröffentlichungstag der Anmeldung:
**05.03.2003 Patentblatt 2003/10**

(73) Patentinhaber: **3M Espe AG**
**82229 Seefeld (DE)**

(72) Erfinder:
- **NOWAK, Reinhold**
  **82276 Adelshofen (DE)**
- **ZECH, Joachim**
  **86916 Kaufering (DE)**
- **BISSINGER, Peter**
  **86911 Diessen (DE)**
- **WANEK, Erich**
  **86916 Kaufering (DE)**
- **ECKHARDT, Gunther**
  **06231 Bad Dürrenberg (DE)**
- **LECHNER, Günther**
  **82237 Wörthsee (DE)**

(56) Entgegenhaltungen:
**EP-A- 0 982 041**
**WO-A-00/47165**
**US-A- 3 159 600**

- **PATENT ABSTRACTS OF JAPAN vol. 009, no. 237 (C-305), 24. September 1985 (1985-09-24) & JP 60 094486 A (DAINIPPON INSATSU KK;OTHERS: 01), 27. Mai 1985 (1985-05-27) & DATABASE WPI/DERWENT [Online] An 1985-163004,**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 287 057 B1

## Beschreibung

Die Erfindung betrifft Aziridinosilikone sowie deren Verwendung, insbesondere in dentalen Zubereitungen.

Unter Aziridinosilikonen im Sinne dieser Erfindung sind ölige oder harzartige polymere Siloxane gemeint, die mit Aziridinogruppen funktionalisiert sind (analog der Bezeichnung "Vinyl- oder Hydrido-Silikonöle" in A. Tomanek, Silicone & Technik, Hanser 1990, S. 37).

Hochpräzise elastische Abformmaterialien, die sich durch hohe Abformgenauigkeit, hohe Formbeständigkeit und gute Detailwiedergabe auszeichnen, sind beispielsweise Materialien auf der Basis von Agar-Agar, Polysulfiden, Polyethern oder additionsvernetzenden Silikonen.

Bei den additionsvernetzenden Silikonabformmaterialien wird die Härtung durch Reaktion eines Polysiloxans mit Vinylendgruppen mit einem Polysiloxan mit Si-H-Gruppen mittels Platinkatalysatoren erreicht. Die so erhaltenen Abdrücke zeichnen sich durch sehr gute elastische Eigenschaften und hohe Lagerbeständigkeiten aus. Nachteilig bei diesen Materialien ist schon immer die geringe Hydrophilie gewesen, die zu geringer Zeichnungsschärfe aufgrund eines schlechten Anfließvermögens führt.

Zur Verbesserung des hydrophilen Verhaltens von Silikonabdruckmaterialien werden den additionsvemetzenden Silikonabdruckmaterialien hydrophilierende Zusätze hinzu gefügt. Die so erzielte bessere Benetzbarkeit ist jedoch auch mit einer erhöhten Wasseraufnahme beim Kontakt mit feuchten Medien verbunden, was eine verschlechterte Dimensionsstabilität und verstärkte Wasserstoffentwicklung zur Folge haben kann.

Bei den reinen Polyethermaterialien werden aziridinhaltige Substanzen polymerisiert, wie sie in den US-A-34 53 242 und 40 93 555 oder auch in der DE-A-43 06 997 beschrieben sind. Zur Initiierung der Polymerisation sind beispielsweise die aus der US-A-41 67 618 bekannten Sulfoniumsalze gut geeignet. So hergestellte Polyetherabformmaterialien weisen natürliche hydrophile Eigenschaften auf.

Es gab verschiedene Ansätze, die hydrophilen Eigenschaften der Polyether mit den guten elastischen Eigenschaften der Silikone zu kombinieren:

In der DE-A-37 41 575, DE-A-40 19 249, DE-A-40 10 281 sowie in der DE-A-38 38 587 sind Massen auf der Basis einer platinkatalysierten Additionsreaktion einer Si-H-Komponente mit einem ungesättigten Polyether beschrieben. Der ungesättigte Polyether ist im Gegensatz zu additionsvernetzenden Silikonen in der Regel der Hauptbestandteil, welcher der Matrix eine hydrophile Charakteristik verleiht.

In der DE-A-40 19 249 sind härtbare Massen beschrieben, die neben ungesättigten Polyethem mit endständigen Alkenylresten auch die Umsetzungsprodukte solcher substituierter Polyether mit Oligosiloxanen mit mindestens zwei Si-H-Gruppen im Molekül sowie Platinkatalysatoren als Hauptbestandteile enthalten.

Zur Erlangung ausreichender Lagerfähigkeit ist es notwendig, die reaktiven Bestandteile räumlich voneinander zu trennen. Hierbei können die Si-H-Verbindung und der für eine Aushärtung bei Raumtemperatur erforderliche Platinkatalysator nicht in einer Paste vereinigt werden, da es zur Zersetzung der Si-H-Verbindung kommt.

Im Zuge einer Lagerung über einen Zeitraum von mehreren Wochen bis Monaten zeigt sich jedoch das Problem, dass auch eine Katalysatorkomponente, bei der der Platinkatalysator mit dem ungesättigten Polyether zusammen vorliegt, eine nicht befriedigende Lagerstabilität aufweist.

Für den Zahnarzt oder den Techniker besteht allerdings die Notwendigkeit, eine Abformmasse zur Verfügung zu haben, die lagerfähig ist und deren Anwendbarkeit über einen Zeitraum von mehreren Monaten bis Jahren gewährleistet ist.

In DE-A-40 10 281 wird daher ein Zusatz von Antioxidantien zur Erhöhung der Lagerstabilität vorgeschlagen. Jedoch wird auch dadurch nur eine nicht zufriedenstellende Langzeit-Lagerstabilität erreicht.

In der DE-A-197 19 438 werden additionsvemetzende Polyether-Abformmaterialien beschrieben, die sich durch eine gute Lagerstabilität der Katalysatorkomponente sowie der Basiskomponente als auch der ausgehärteten Dentalmasse auszeichnen. Nachteilig an diesen Dentalmassen ist das niedrige Niveau der mechanischen Eigenschaften, so dass sie nur eingeschränkt für die dentale Abformung einsetzbar sind.

Aufgabe der vorliegenden Erfindung ist es, härtbare Massen bereitzustellen, die die Nachteile des Standes der Technik nicht aufweisen.

Diese Aufgabe wurde gelöst durch N-Alkylaziridinosilikone als Basis für die Herstellung von Dentalmassen, wie sie in den Ansprüchen beschrieben sind.

Die Einzelkomponenten der daraus hergestellten Massen weisen eine gute Lagerbeständigkeit auf. Beispielsweise wird bei der Verwendung als Dentalmassen eine hohe Abformgenauigkeit erzielt. Im ausgehärteten Zustand zeichnen sich diese durch gute mechanische Eigenschaften aus.

Die erfindungsgemäßen N-Alkylaziridinosilikone haben die nachfolgend dargestellte allgemeine Struktur nach Formel (1):

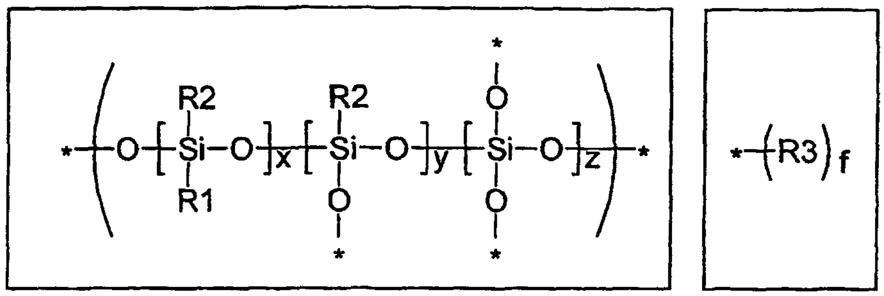

(1.1) (1.2)

(1)

wobei:

R1 = H, $C_1$-$C_{12}$-Alkyl, $C_2$-$C_{12}$-Alkenyl, $C_2$-$C_{12}$-Alkinyl, $C_7$-$C_{15}$-Alkaryl, $C_7$-$C_{15}$-Aralkyl, $C_3$-$C_{12}$-Cycloalkyl bedeutet und diese Reste teilweise, ganz oder gemischt mit Cl oder F substituiert sein können und/oder 0 bis 5 Heteroatome aus der Gruppe O, N, S enthalten können und bevorzugt H, Methyl, Ethyl, Ethenyl, Propenyl, Phenyl, Tolyl, 2-Ethylphenyl, Cyclohexenyl verwendet werden,

R2 = ein Rest aus der Auswahl R1 und/oder R4 bedeutet, und

R3 = $SiR1_3$ oder $SiR1_2R4$,

mit R4 =

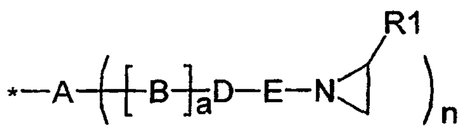

(2)

und

A = ein (n+1)-fach radikalischer gesättigter, ungesättigter oder aromatischer, linearer, verzweigter oder cyclischer Kohlenwasserstoffrest, der 0 bis 5 Heteroatome aus der Gruppe O, N, S enthalten kann und 1 bis 18 Kohlenstoffatome, bevorzugt 1 bis 12 Kohlenstoffatome umfasst,

B = ausgewählt aus der Gruppe O, S, NR1,

D = ausgewählt aus der Gruppe C(O)O, C(O)NR1, C(O), C(O)C(O), $C(O)(CH_2)_m(C(O)$, C(S)NR1, $CH_2$,

E = ein 2-fach radikalischer gesättigter oder ungesättigter, linearer, verzweigter oder cyclischer Kohlenwasserstoffrest, der 0 bis 5 Heteroatome aus der Gruppe O, N, S enthalten kann und 0 bis 18 Kohlenstoffatome, bevorzugt 1 bis 12 Kohlenstoffatome umfasst,

a = 0 oder 1,

f = eine ganze Zahl von 2 bis 1000, bevorzugt 2 bis 100, besonders bevorzugt 2 bis 50,

n, m = eine ganze Zahl von 1 bis 10, bevorzugt 1 bis 5, besonders bevorzugt 1 bis 3,

sowie

x, y, z jeweils entweder 0 oder ganze Zahlen darstellen, deren Summe zwischen 1 und 10.000, bevorzugt zwischen 1 und 1.000 und besonders bevorzugt zwischen 10 und 500 liegen soll, wobei die mittlere Molmasse $M_n$ der Produkte bevorzugt zwischen 500 und 50.000 und besonders bevorzugt zwischen 1.000 und 20.000 liegen kann,

mit den Einschränkungen, dass, wenn x größer 0 ist, y oder z kleiner oder gleich x sind, bevorzugt kleiner oder gleich 0,05 mal x und besonders bevorzugt 0,02 mal x sein soll.

Das Symbol "*" in der Formel (1.2) bedeutet, dass die so markierte Valenz mit den mit "*" markierten Positionen des Fragments (1.1) verknüpft wird.

Von den jeweils x Resten von R2 können bis zu 0,5 mal x Reste R4 bedeuten, die anderen bedeuten R1.

Im Fall von y + z = 0 liegen lineare Polysiloxane, im Fall von y + z > 0 verzweigte Polysiloxane vor, wobei Formel (1) insofern zu verstehen ist, als das Polymer in diesem Falle sowohl D-, als auch T- und / oder Q-Einheiten im Sinne der Silikon-Nomenklatur enthalten kann, die im Molekül an beliebiger Stelle stehen können und an den Anknüpfungspunkten der T- und Q-Einheiten beliebig lange D-Ketten aufweisen, deren summarische Länge x D-Einheiten beträgt.

Jede Nennung des Restes R1 bedeutet lediglich die Entnahme aus der unter R1 getroffenen Auswahl, verschiedene Nennungen können an jedem unterschiedlichen Substitutionspunkt und auch an jeder Wiederholungseinheit einer polymeren Formel, unterschiedliche Reste R1 bedeuten. Das bedeutet, dass mit der Struktureinheit $-(SiOR1R2)_x-$ sowohl Homopolymere mit einem definierten R1 und R2 als auch Copolymere von Silikonen unterschiedlicher Reste R1 und R2 gemeint sind, wobei x jedoch die Anzahl aller damit umfassten Siliciumatome ohne Ansehen der getroffenen Auswahl an Resten R1 und R2 bedeutet. Analoges gilt sinngemäß auch für den Faktor-$(SiR2(O^*))_y$- und den Rest R3.

Im allgemeinen sind keine polymereinheitlichen Polysiloxan-Grundketten vorhanden. In Abhängigkeit vom Herstellungsverfahren kann die Polydispersität ($\overline{M}_w/\overline{M}_n$) 1,1 bis 20 und bevorzugt 1,2 bis 10 betragen.

Die erfindungsgemäßen N-Alkylaziridinosilikone besitzen Molmassen im Bereich von 500 bis 50.000 g/Mol, bevorzugt von 1.000 bis 30.000 g/Mol und besonders bevorzugt im Bereich von 3.000 bis 20.000 g/Mol. Sie besitzen mindestens eine und bis zu zehn N-Alkylaziridinogruppen im Molekül.

Zur Einstellung der jeweils gewünschten Netzwerkstruktur können die verwendeten N-Alkylaziridinosilikone stark unterschiedliche Aziridinoäquivalentmassen besitzen, wobei der Bereich von 250 bis 25.000 g/Äquivalent und besonders von 400 bis 10.000 g/Äquivalent bevorzugt ist.

Bevorzugte Beispiele für die erfindungsgemäßen N-Alkylaziridinosilikone sind solche, die durch Kombination von bevorzugten Vertretern der Formel (1) und Formel (2) im Sinne der allgemeinen Formeln erhalten werden.

Die Anknüpfung von Vertretern von Formel (2) an solche von Formel (1) wird häufig mittels Hydrosilylierungsreaktion bewerkstelligt. Diese führt wie dem Fachmann bekannt ist (Bogdan Marciniec "Comprehensive Handbook on Hydrosilylation") im allgemeinen zu Gemischen des α- und β-Adduktes. Bei allen Formelbeispielen wird das β-Addukt gezeigt, wobei jedoch auch immer das α-Addukt mit umfasst sei.

Bevorzugte Vertreter der Formel (1) sind aus der Gruppe lineare Siloxane, kammartige Silikone, T-verzweigte Siloxane sowie MQ-Silikon-Harze entnommen. Bevorzugte Vertreter dieser einzelnen Gruppen sind:

- Lineare Siloxane

  Gemeint sind für die folgenden Formeln der endständig aziridinofunktionalisierten Silikone auch alle Copolymere mit Dimethylsiloxan.

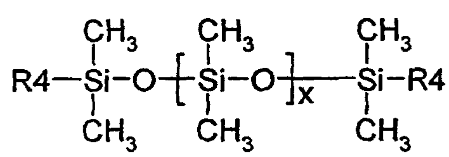

aus: R1 = Methyl, R2 = R1, x = 0 bis 500, R3 = $SiMe_2R4$
(R4 siehe Formel (2)), y, z = 0, f = 2

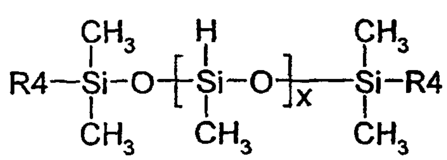

aus: R1 = Methyl, R2 = H, x = 0 bis 500, R3 = $SiMe_2R4$
(R4 siehe Formel (2)), y, z = 0, f = 2

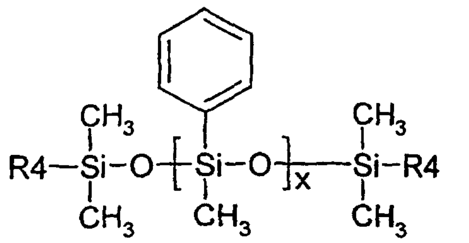

aus: R1 = Methyl, Phenyl, R2 = R1, x = 0 bis 500, R3 = $SiMe_2R4$ (R4 siehe Formel (2)), y, z = 0, f = 2

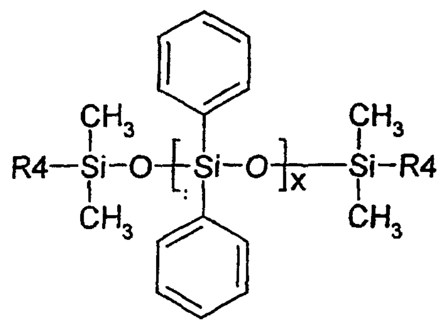

aus: R1 = Methyl, Phenyl, R2 = R1, x = 0 bis 500, R3 = $SiMe_2R4$ (R4 siehe Formel (2)), y, z = 0, f = 2

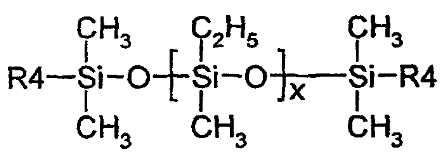

aus: R1 = Methyl, Ethyl, R2 = R1, x = 0 bis 500, R3 = $SiMe_2R4$ (R4 siehe Formel (2)), y, z = 0, f = 2

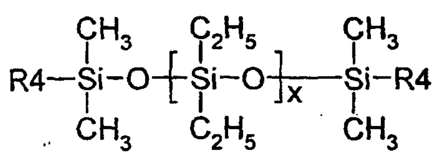

aus: R1 = Methyl, Ethyl, R2 = R1, x = 0 bis 500, R3 = $SiMe_2R4$ (R4 siehe Formel (2)), y, z = 0, f = 2

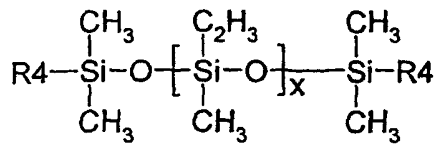

aus: R1 = Methyl, Vinyl, R2 = R1, x = 0 bis 500, R3 = $SiMe_2R4$ (R4 siehe Formel (2)), y, z = 0, f = 2

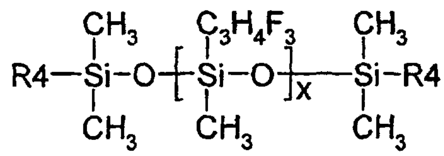

aus: R1 = Methyl, 3,3,3-Trifluorpropyl, R2 = R1, x = 0 bis 500, R3 = $SiMe_2R4$ (R4 siehe Formel (2)), y, z = 0, f = 2

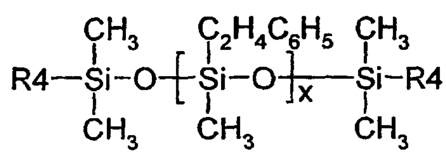

aus: R1 = Methyl, Phenylethylenyl, R2 = R1, x = 0 bis 500, R3 = $SiMe_2R4$ (R4 siehe Formel (2)), y, z = 0, f = 2

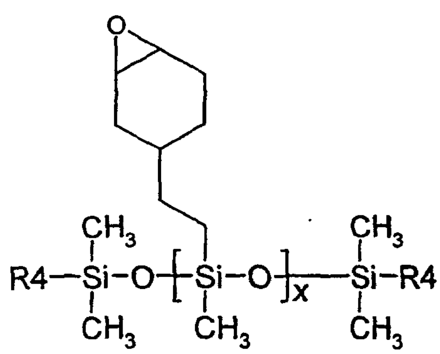

aus: R1 = Methyl, Epoxycyclohexylethylenyl, R2 = R1, x = 0 bis 500, R3 = $SiMe_2R4$, (R4 siehe Formel (2)), y, z = 0, f = 2

- Kammartige Silikone

  Gemeint sind für die folgenden Formeln der seitenständig aziridinofunktionalisierten Silikone auch alle Copolymere mit Dimethylsiloxan, wobei sich die Zahl x der funktionalisierten Siloxaneinheiten dabei um den Anteil der Dimethylsiloxaneinheiten reduziert.

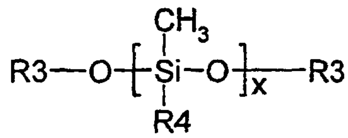

aus: R1 = Methyl, R2 = R4, x = 0 bis 500, R3 = $SiMe_2R4$ (R4 siehe Formel (2)) oder $SiR1_3$ (R1 = Methyl); y, z = 0, f = 1 bis 100

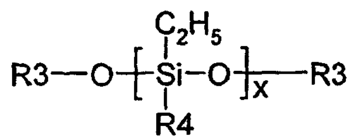

aus: R1 = Ethyl, R2 = R4, x = 0 bis 500, R3 = $SiMe_2R4$ (R4 siehe Formel (2)) oder $SiR1_3$ (R1 = Methyl); y, z = 0, f = 1 bis 100

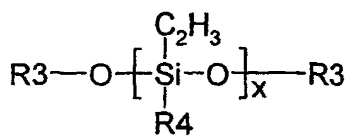

aus: R1 = Vinyl, R2 = R4, x = 0 bis 500, R3 = $SiMe_2R4$ (R4 siehe Formel (2)) oder $SiR1_3$ (R1 = Methyl); y, z = 0, f = 1 bis 100

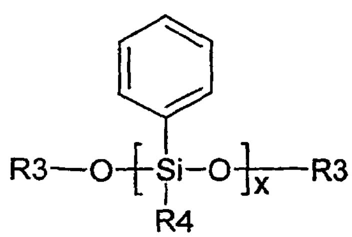

aus: R1 = Phenyl, R2 = R4, x = 0 bis 500, R3 = $SiMe_2R4$ (R4 siehe Formel (2)) oder $SiR1_3$ (R1 = Methyl), y, z = 0, f = 1 bis 100

- T-verzweigte Siloxane

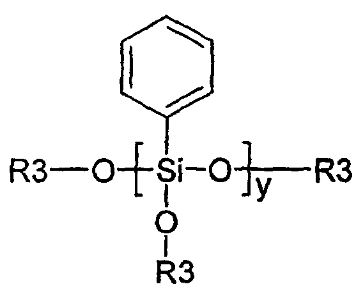

aus: R1 = Methyl, R2 = R4, x = 0 , R3 = $SiMe_2R4$ (R4 siehe Formel (2)) oder $SiR1_3$(R1 = Methyl); y = 1 - 500, z = 0, f = 1 bis 100

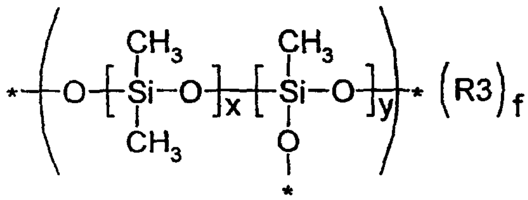

aus: R1 = Methyl, R2 = R1, x = 0 - 500, y = 1 - 25; R3 = $SiMe_2R4$ (R4 siehe Formel (2)) oder $SiR1_3$ (R1 = Methyl); z = 0, f = 1 bis 100

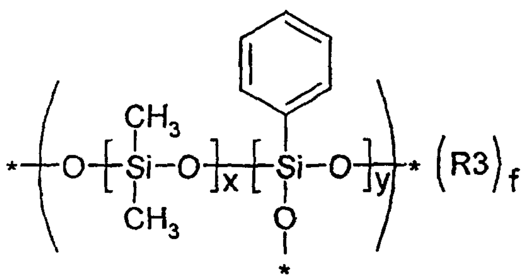

aus: R1 = Methyl, R2 = Phenyl, x = 0 - 500, y = 1 - 25; R3 = $SiMe_2R4$ (R4 siehe Formel (2)) oder $SiR1_3$ (R1 = Methyl); z = 0, f = 1 bis 100

- MQ-Silikon-Harze

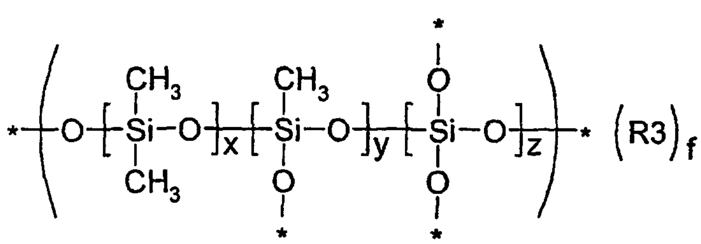

aus: R1 = Methyl, R2 = R1, x = 0 bis 500, y = 0 bis 25; z = 1 bis 25; R3 = $SiMe_2R4$ (R4 siehe Formel (2)) oder $SiR1_3$ (R1 = Methyl); f = 1 bis 100

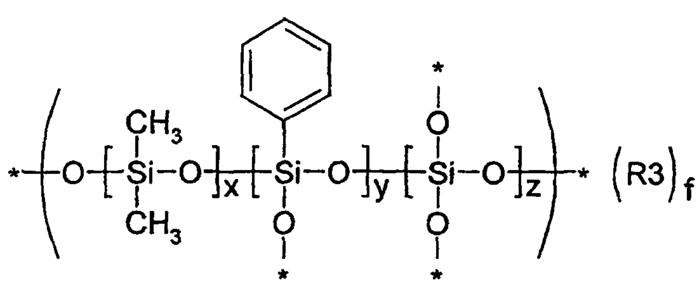

aus: R1 = Methyl, R2 = Phenyl, x = 0 bis 500, y = 0 bis 25; z = 1 bis 25; R3 = $SiMe_2R4$ (R4 siehe Formel (2)) oder $SiR1_3$ (R1 = Methyl); f = 1 bis 100

Bevorzugte Vertreter der Formel (2) sind, wobei neben den N-Alkyl-ethylenimin-Derivaten auch die N-Alkyl-propylenimin-Derivate gemeint sind:

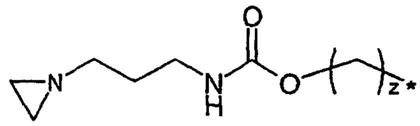

z = 1 - 10

aus: A = $(CH_2)_z$; B = O; D = C(O)NR1 (mit R1 = H); E = 1,3-propandiyl; a = 1; n = 1

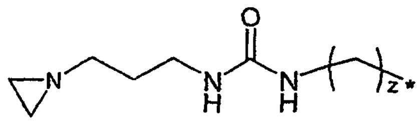

z = 1 - 10

aus: A = $(CH_2)_z$; B = NH; D = C(O)NR1 (mit R1 = H); E = 1,3-propandiyl; a=1;n=1

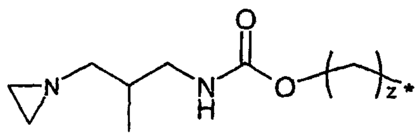

z = 1 - 10

aus: A = $(CH_2)_z$; B = O; D = C(O)NR1 (mit R1 = H); E = 2-methyl-1,3-propandiyl; a = 1; n = 1

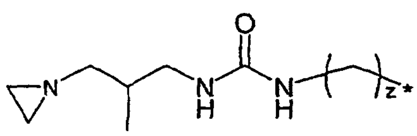

z = 1 - 10

aus: A = $(CH_2)_z$; B = NH; D = C(O)NR1 (mit R1 = H); E = 2-methyl-1,3-propandiyl; a = 1; n = 1

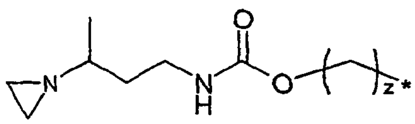

z = 1 - 10

aus: A = $(CH_2)_z$; B = O; D = C(O)NR1 (mit R1 = H); E = 1,3-butandiyl; a = 1; n = 1

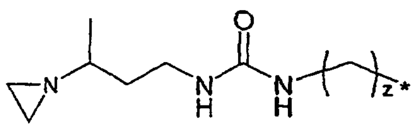

z = 1 - 10

aus: A = $(CH_2)_z$; B = NR1 (R1 = H); D = C(O)NR1 (mit R1 = H); E = 1,3-hutandiyl; a = 1; n = 1

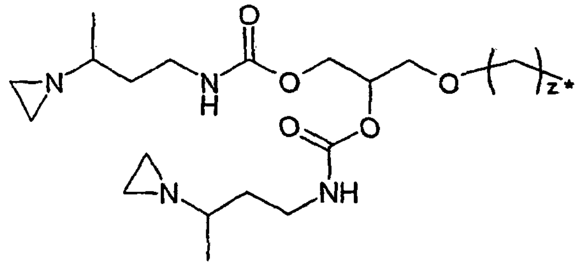

z = 1 - 10

aus: A = 3-oxa-heptan-1,2,7-triyl (für z=3); B = O; D = C(O)NR1 (mit R1 = H); E = 1,3-butandiyl; a = 1; n = 2

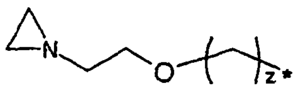

z = 1 - 10

aus: A = $(CH_2)_z$; B = O; D = $CH_2$; E = methandiyl; a = 1; n = 1

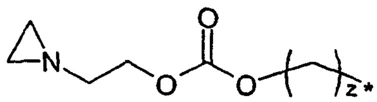

z = 1 - 10

aus: A = $(CH_2)_z$; B = O; D = C(O)O; E = 1,2-ethandiyl; a = 1; n = 1

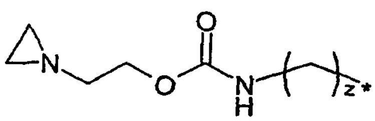

z = 1 - 10

aus: A = $(CH_2)_z$; B = O; D = C(O)O; E = 1,2-ethandiyl; a = 1; n = 1

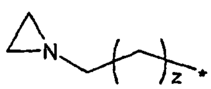

z = 1 - 10

aus: A = $(CH_2)_{z+1}$; D = $CH_2$; E = $CH_2$; a = 0; n = 1

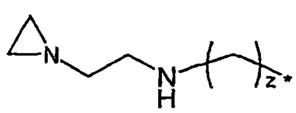

z = 1 - 10

aus: A = $(CH_2)_z$; B = NH; D = $CH_2$; E = 1,2-ethandiyl; a = 1; n = 1

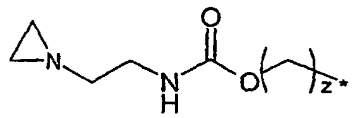

z = 1 - 10

aus: A = $(CH_2)_z$; B = NH; D = C(O)O; E = 1,2-ethandiyl; a = 1; n = 1

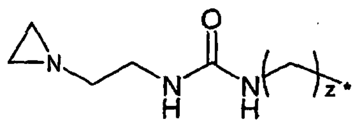

z = 1 - 10

aus: A = $(CH_2)_z$; B = NH; D = C(O)NH; E = 1,2-ethandiyl; a = 1; n = 1

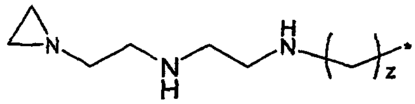

z = 1 - 10

aus: A = $(CH_2)_z$; B = NH; D = $CH_2$; E = 2-aza-1,4-butandiyl; a = 1; n = 1

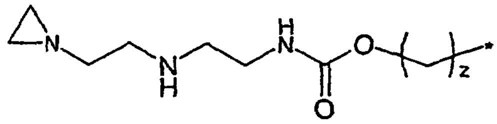

z = 1 - 10

aus: A = $(CH_2)_z$; B = NH; D = C(O)O; E = 2-aza-1,4-butandiyl; a = 1; n = 1

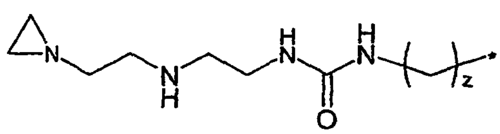

z = 1 - 10

aus: A = $(CH_2)_z$; B = NH; D = C(O)NH; E = 2-aza-1,4-butandiyl; a = 1; n = 1

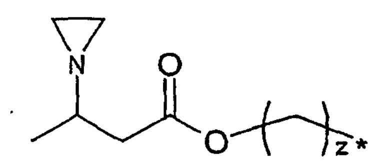

z = 1 - 10

aus: A = $(CH_2)_z$; B = O; D = C(O); E = 2-methyt-1,2-propandiyl; a = 1; n = 1

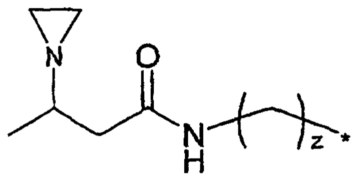

z = 1 - 10

aus: A = $(CH_2)_z$; B = NH; D = C(O); E = 2-methyl-1,2-propandiyl; a = 1; n = 1

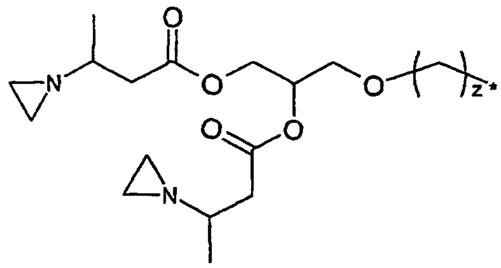

z = 1 - 10

aus: A = 3-oxa-heptan-1,2,7-triyl; B = O; D = C(O);
E = 2-methyl-1,2-propandiyl; a = 1; n = 2

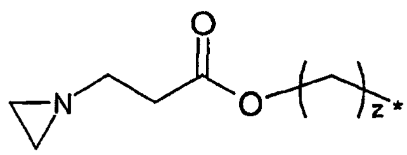

z = 1 - 10

aus: A = $(CH_2)_z$; B = O; D = C(O); E = 1,2-ethandiyl; a = 1; n = 1

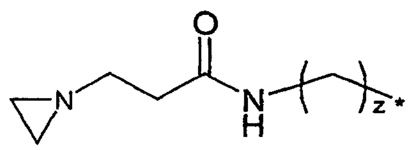

z = 1 - 10

aus: A = $(CH_2)_z$; B = NH; D = C(O); E = 1,2-ethandiyl; a = 1; n = 1

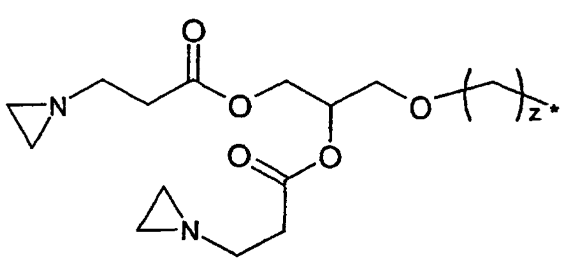

z = 1 - 10

aus: A = 3-oxa-heptan-1,2,7-triyl; B = O; D = C(O); E = 1,2-ethandiyl;
a=1;n=2

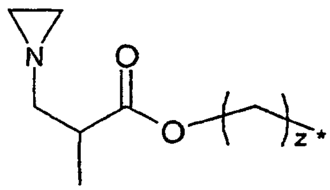

z = 1 - 10

aus: A = $(CH_2)_z$; B = O; D = C(O); E = 1-methyl-1,2-propandiyl; a = 1; n = 1

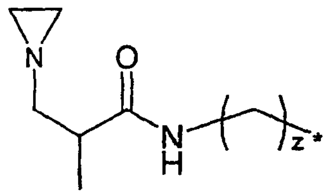

z = 1 - 10

aus: A = $(CH_2)_z$; B = NH; D = C(O); E = 1-methyl-1,2-propandiyl; a = 1; n = 1

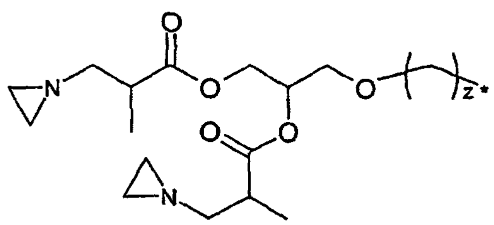

z = 1 - 10

aus: A = 3-oxa-heptan-1,2,7-triyl; B = O; D = C(O);
E = 1,-methyl-1,2-propandiyl; a = 1; n = 2

Prinzipiell wird bei der Herstellung der erfindungsgemäßen N-Alkylaziridinosilikone so verfahren, dass ein käuflich erhältliches funktionalisiertes Polysiloxan in einem oder mehreren Schritten zum Aziridinosilikon umgesetzt wird. Statt die käuflich erhältlichen funktionalisierten Polysiloxane als solche einzusetzen, können diese auch in sich, als Mischung und/oder als Mischung mit Silikonrohstoffen durch Äquilibrierung, Polymerisation, Copolymerisation, Depolymerisation etc. in bekannter Art (siehe P. Kochs in Houben-Weyl Bd. E20 S. 2219 ff.) verändert und erst danach zum Aziridinosilikon funktionalisiert werden. Eine Vielzahl von Vertretern solcher funktionalisierten Silikonöle können zum Beispiel im Katalog "Reactive Silicones" der Firma Gelest gefunden werden. Sie können jedoch auch nach literaturbekannten Vorschriften selbst hergestellt werden (H. R. Kricheldorf (Ed.) "Silicon in Polymer Synthesis" Springer 1996, Chapt. 3). Besonders geeignet zur Funktionalisierung sind Hydrido-, Acryl-, Methacryl-, Vinyl-, Hydroxyalkyl- und Aminoalkyl-funktionalisierte Siloxane.

Daraus können beispielsweise über Chloroxalate oder Chlorformiate Aziridinosilikone hergestellt werden, wobei in manchen Fällen aus den Chloroxalaten oder -formiaten intermediär aktivierte Amide, wie Imidazolide hergestellt werden. Als Aziridinokomponente kommen beispielsweise Aziridinoethanol (Acros) oder andere hydroxy- oder aminofunktionelle Aziridinoverbindungen in Frage. Ein anderer Weg besteht darin, Aziridin an aktivierte Doppelbindungen von entsprechend funktionalisierten Silikonen anzulagern.

Neben diesen Methoden zur Synthese von Aziridinoverbindungen, finden sich in den folgenden Monographien sowie in den darin zitierten Literaturstellen eine Vielzahl von möglichen Synthesevarianten zum Aufbau und zur Derivatisierung oder Modifizierung von Aziridinoverbinungen. Die bevorzugten Vertreter der Aziridinosilikone sowie nötigenfalls deren Vorstufen sind nach den dort beschriebenen Vorgehensweisen darstellbar, wobei einige dieser Methoden vom Fachmann sinnvoll anzupassen sind: R.C. Elderfield, "Heterocyclic Compounds" Vol. 1., S. 61-77 Wiley 1950; Houben-Weyl "Methoden der Organischen Chemie", Bd. XI/2 S. 223-264, Thieme 1958; O.C. Dermer, G.E. Ham, "Ethylenimine and other Aziridines", insbesondere S. 106-205 sowie S. 340-393, Academic Press 1969; Houben-Weyl "Methoden der Organischen Chemie", Bd. E16c S. 370-667, Thieme 1992; Ulmanns Encyclopedia of Industrial Chemistry 5th Ed. Vol. A3 S. 239-243.

Weiterhin sind Gegenstand der Erfindung härtbare Massen auf der Basis der erfindungsgemäßen N-Alkylaziridinosilikone, insbesondere zweikomponentige Dentalmassen, wobei diese nach der Mischung der Komponenten, jeweils bezogen auf 100 Masseteile, enthalten:

(A) 30 bis 97, bevorzugt 40 bis 89, besonders bevorzugt 45 bis 80,5 Masseteile von mindestens einem N-Alkylaziridinosilikon mit Molmassen im Bereich von 500 bis 50.000 g / Mol und Aziridinoäquivalentmassen im Bereich von 250 bis 25.000 g / Äquivalent

(B) 1 bis 10, bevorzugt 1 bis 5, besonders bevorzugt 1,5 bis 3 Masseteile von Startersubstanzen, die geeignet sind, die Aushärtung der N-Alkylaziridinosilikone zu bewirken,

(C) 1 bis 35, bevorzugt 5 bis 25, besonders bevorzugt 8 bis 20 Masseteile von organischen Verdünnungsmitteln,

(D) 1 bis 50, bevorzugt 5 bis 40, besonders bevorzugt 10 bis 30 Masseteile von Modifikatoren, einschließlich Füllstoffen, Farbstoffen, Pigmenten, Thixotropiemitteln, Fließverbesserern, polymeren Eindickern, oberflächenaktiven Substanzen, Geruchsstoffen und Geschmacksstoffen.

Die durch homogene Mischung der Katalysatorkomponente und der Basiskomponente erzeugte Zubereitung besitzt beispielsweise eine Verarbeitungszeit bei Raumtemperatur zwischen 0,5 bis 10 Minuten und die so hergestellte Zubereitung härtet beispielsweise in einem Temperaturbereich von 23 bis 36° C innerhalb einer Zeitspanne von einer bis 20 Minuten zu einer elastisch deformierbaren Masse mit einer Shore A-Härte von mindestens 20 aus.

Im Falle von zweikomponentigen Massen werden die Bestandteile (A) bis (D) vor der Mischung auf die Basis- und die Katalysatorkomponente so aufgeteilt, dass der Bestandteil (A) vollständig in der Basiskomponente und der Bestandteil (B) vollständig in der Katalysatorkomponente enthalten ist. Die Bestandteile (C) und (D) können anteilig in den Komponenten vorliegen.

Die Anteile der einzelnen Bestandteile (A) bis (D) sind innerhalb der angegebenen Grenzen so einzustellen, dass eine günstige Verarbeitbarkeit hinsichtlich Mischungsverhältnis und Fließverhalten gewährleistet ist und die Kriterien für die gewünschte Verarbeitungszeit, die Aushärtungszeit und die mechanischen Eigenschaften der ausgehärteten Masse eingehalten werden.

Das Mischungsverhältnis kann in einem weiten Bereich über die Zusammensetzung der beiden Komponenten eingestellt werden, wobei sich Mischungsverhältnisse von Katalysator- zu Basiskomponente von 1 : 1 bis 1 : 5 als besonders praktikabel erwiesen haben.

Bestandteil (A) enthält die erfindungsgemäßen N-Alkylaziridinosilikone. Die Verwendung von Gemischen von N-Alkylaziridinosilikonen mit unterschiedlichen Molmassen und Aziridinoäquivalentmassen ist möglich und wird zur Einstellung der Eigenschaften der Dentalmassen genutzt.

Als Startersubstanz gemäß Bestandteil (B) der gemischten Zubereitung kommen eine Reihe von Verbindungen in Betracht, wenn sie die Kriterien hinsichtlich Abbindegeschwindigkeit und resultierenden Elastomereigenschaften erfüllen.

So sind für die Verwendung in zweikomponentigen Abformmassen, die auf den vorstehend beschriebenen Polyetherderivaten beruhen, solche Startersubstanzen geeignet, die eine Aushärtung der gemischten Zubereitung in einem Zeitraum von 1 bis 20 Minuten zu einem elastischen Festkörper ermöglicht, wobei dieser Festkörper die Anforderungen an eine elastische Abformmasse gemäß DIN / EN 2482 erfüllt und eine Shore A-Härte (DIN 53505) von mindestens 20 nach 24 Stunden Lagerzeit besitzt.

Als Starter gemäß Bestandteil (B) der Katatysatorkomponente können viele der bekannten Starter eingesetzt werden. Zweckmäßigerweise verwendet man solche Starter bzw. Startersysteme, die eine einfache Einstellung des Aushärtungsverlaufs zulassen, keine Nebenwirkungen erzeugen und die reproduzierbare Erreichung der erforderlichen Niveaus der mechanischen Eigenschaften ermöglichen.

In der DE-C-914 325 wird die Verwendung von Oxonium-, Ammonium- und Sulfoniumsalzen als Startersubstanzen vorgeschlagen.

Eine zusammenfassende Darstellung der für die Aushärtung von N-Alkylaziridinoverbindungen verwendeten Startersubstanzen ist in O. C. DERMER, G. E. HAM, "Ethylenimine and other Aziridines" Academic Press (1969) enthalten.

Als prinzipiell geeignete Polymerisationsauslöser haben sich demnach eine große Anzahl von Verbindungsklassen und Verbindungen erwiesen. In der praktischen Anwendung der kationischen Polymerisation von Aziridinopolyethem ist es aber sehr schwierig, den gewünschten Abbindungsverlauf mit ausreichend langer Verarbeitungszeit und schneller Endaushärtung einzustellen. Dieses Ziel kann durch die Verwendung von speziellen Trisalkylsulfoniumsalzen erreicht werden, wie sie beispielsweise in der EP-A-0 110 429 beschrieben sind.

Unter Verwendung von speziellen Trisalkylsulfoniumsalzen sind die Kriterien der die Härtungsgeschwindigkeit und der Eigenschaften des elastischen Festkörpers prinzipiell erreichbar.

In der Patentanmeldung DE-100 18 918 werden Starter beschrieben, die der Katalysatorkomponente einen lediglich geringen Säuregrad verleihen und die eine gut einstellbare, relativ lange Verarbeitungszeit nach erfolgter

Mischung von Basiskomponente und Katalysatorkomponente ermöglichen.

Startersysteme dieses Typs sind geeignet, die erfindungsgemäßen Basispasten in der notwendigen Geschwindigkeit auszuhärten. Durch ihre Verwendung sind die gewünschten Eigenschaften des elastischen Festkörpers erreichbar.

Die Patentanmeldung DE- 19942459 beschreibt Elastomermassen mit verbesserter Katalysatorkomponente, die sich durch eine erhöhte Dehnbarkeit auszeichnen. Gemäß dieser Erfindung werden Borsäurekomplexe als Starter eingesetzt. Diese Starter haben sich für die Aushärtung der N-Alkylaziridinopolyether gemäß vorliegender Erfindung besonders bewährt und werden mit Vorteilen gegenüber anderen Startersystemen eingesetzt.

Als organisches Verdünnungsmittel entsprechend Bestandteil (C) werden Polyetherpolyole, wie Polypropylenglykole oder Mischpolyetherole mit Tetrahydrofuran- und/oder Ethylenoxid- und/oder Propylenoxid-Einheiten, Polyesterpolyole, wie Polycaprolactondiole und Polycaprolactontriole, Polycarbonatdiole, aliphatische Ester, Öle, Fette, Wachse, aliphatische Kohlenwasserstoffe, araliphatische Kohlenwasserstoffe sowie ein- oder mehrfunktionelle Ester von mehrwertigen Säuren wie beispielsweise Phthalsäure oder Zitronensäure oder Ester oder Amide von Alkylsulfonsäuren und Arylsulfonsäuren verwendet.

Weiterhin sind flüssige Organosiloxane, wie Polydimethylsiloxane unterschiedlicher Kettenlänge einsetzbar. Mit Vorteil einzusetzende Verbindungen gemäß diesem Bestandteil genügen den nachfolgenden allgemeinen Strukturen:

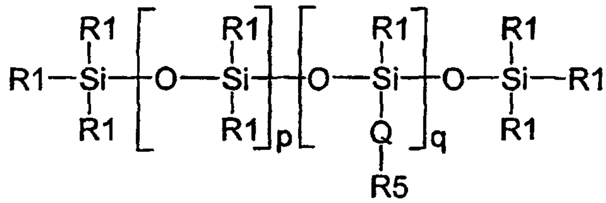

und/oder

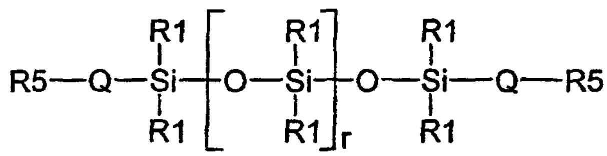

wobei bedeuten:

Q = eine α, ω-radikalische Polyetherkette der Formel (3):

$$- [O\text{-}C_gH_{2g}]_h - \qquad (3);$$

R5 = OH, OR1, O-C(O)-R1, O-C(O)-NHR1;
g = 2 bis 20, bevorzugt 2 bis 10, besonders bevorzugt 2 bis 7;
h = 1 bis 1000, bevorzugt 1 bis 500, besonders bevorzugt 1 bis 200;
p = eine ganze Zahl von 1 bis 1000, bevorzugt 3 bis 500;
q = eine ganze Zahl von 1 bis 50, bevorzugt 3 bis 10;
r = eine ganze Zahl von 1 bis 1000, bevorzugt 1 bis 500.

In Abhängigkeit vom Herstellungsverfahren kann die Polydispersität der Polyetherblöcke 1,1 bis 20 und bevorzugt 2 bis 10 betragen.

Der Polyetherblock kann ein Homopolymer, ein Copolymer oder auch ein Terpolymer sein. Die Mischpolymerisate können alternierend oder statistisch aufgebaut sein bzw. alternierende und statistische Mischpolyetherblöcke besitzen, die ggf. mit Homopolymer-Polyetherblöcken verbunden sind.

Bevorzugte Polyetherblöcke sind Polytetrahydrofuran, Polypropylenoxid, statistische Copolymere aus Ethylenoxid und Tetrahydrofuran, aus Propylenoxid und Tetrahydrofuran, aus Ethylenoxid und Propylenoxid, Blockcopolymere aus Ethylenoxid und Propylenoxid und statistische Terpolymere aus Ethylenoxid, Propylenoxid und Tetrahydrofuran.

Der Katalysatorkomponente wie auch der Basiskomponente werden Modifikatoren, entsprechend dem Bestandteil (D) zugesetzt.

Diese Modifikatoren sind meist feinteilige Füllstoffe, wie Alumosilikate, Fällungskieselsäuren, Quarzmehl, Wollastonit, Glimmermehl und Diatomeenerde, sowie Farbstoffe und Pigmente, deren Zusatz eine bessere Beurteilung der Mischgüte ermöglicht und die Verwechslungsgefahr vermindert, Thixotropiemittel, wie feindisperse Kieselsäuren und andere das Fließverhalten beeinflussende Zusätze wie polymere Eindicker, weiterhin oberflächenaktive Substanzen zur Einstellung des Anfließverhaltens sowie Geruchsstoffe und Geschmacksstoffe.

Die erfindungsgemäßen Massen sind besonders geeignet als dentale Abformmassen, Bissregistriermassen, Modellmaterialien, provisorische Verschlussmassen, Materialien zur Herstellung von provisorischen Kronen und Brükken sowie Dubliermassen.

In Abhängigkeit von der Zusammensetzung der Katalysatorkomponente und der Basiskomponente können sie auch für das Verkleben von Substraten, für das Abdichten, das Beschichten und das Vergießen eingesetzt werden.

Dabei kann die Dosierung der beiden Komponenten nach Sicht, wie über Stranglängenvergleich, nach Gewicht, über vordosierte Packungseinheiten und nachfolgende Handanmischung, aus Doppetkammerkartuschen mit statischem Mischrohr oder mittels Volumendosieranlagen mit nachgeschaltetem statischem oder dynamischem Mischer erfolgen.

Zur Erzielung optimaler Ergebnisse ist eine hohe Mischgüte erforderlich. Dagegen ist die Toleranz des Mischungsverhältnisses im allgemeinen relativ groß und kann beispielsweise bei einem vorgegebenen Verhältnis von Katalysatorkomponente zu Basiskomponente von 1 : 5 den Bereich 0,75 bis 1,25 : 5 umfassen, ohne dass einsatzbeschränkende Eigenschaftsänderungen feststellbar sind.

Bei der zahnmedizinischen Abformung erweist sich das gute Anfließverhaiten an den feuchten Zahn und das feuchte Zahnfleisch sowie die Unempfindlichkeit der Präzision der Abformung gegenüber Speichel und Blut als großer Vorteil.

Es wurde gefunden, dass Abformmassen auf der Basis der erfindungsgemäßen Substanzen sich nicht mit Abformmassen oder Modellmaterialien auf der Basis von A-Silikonen und/oder Polyethem verbinden. Dies eröffnet die Möglichkeit, durch Verwendung der erfindungsgemäßen Substanzen ein Situationsabformmaterial bereitzustellen, bei dem der ausgehärtete Abdruck mit einem A-Silikon ausgegossen werden kann, ohne vorher isoliert werden zu müssen.

Gegenstand der Erfindung sind auch Behältnisse und Mischvorrichtungen, enthaltend die aus den erfindungsgemäßen Zubereitungen hergestellten Massen, insbesondere Dentalmassen, wie Kartuschen, Beutel, Abformlöffel, statische und dynamische Mischer bzw. Mischgeräte.

Die Erfindung wird durch die nachfolgenden Beispiele näher erläutert, ohne dass sie durch diese beschränkt werden soll.

Beispiele:

Synthesebeispiel 1:

Darstellung eines Aziridinosilikons aus einem Methacrylat-terminierten Silikonöl

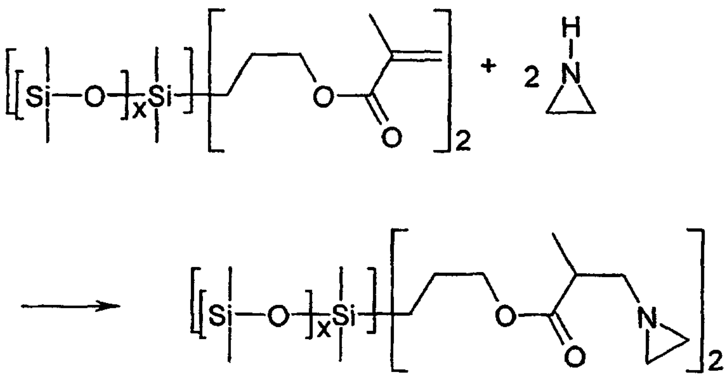

100 g eines Silikonöls DMS-R28 (Gelest) [M = 5000, 0,02 Mol] werden mit 50 ml Toluol sowie 3,44 g Aziridin (Ferak) [M = 43,07, 0,08 Mol] versetzt. Die Mischung wird auf 50° C erhitzt, nach fünf Stunden wird auf 100° C erhöht und solange bei 100° C gehalten, bis die Umsatzkontrolle mittels IR-Spektroskopie das Verschwinden der C=C-Doppelbindung anzeigt. Nach üblicher Aufarbeitung und Produktisolierung verbleiben 100 g eines klaren, farblosen Aziridinosilikons.

Synthesebeispiel 2:

Darstellung eines Aziridinosilikons aus einem Acrylat-terminierten Silikonöl

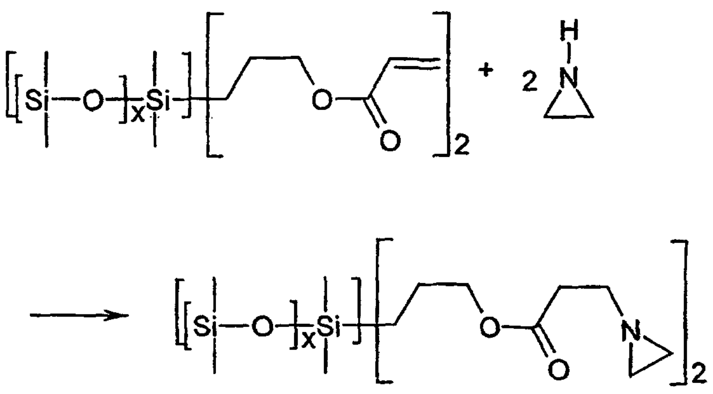

100 g eines Silikonöls DMS-U22 (Gelest) [M = 1100, 0,091 Mol] werden mit 50 ml Toluol versetzt. Bei 30° C tropft man 31,3 g Aziridin [M = 43,07, 0,73 Mol] zu. Die Mischung wird auf 50° C erhitzt, nach fünf Stunden wird auf 100° C erhöht und solange bei 100° C gehalten, bis die Umsatzkontrolle mittels IR-Spektroskopie das Verschwinden der C=C-Doppelbindung anzeigt. Nach üblicher Aufarbeitung und Produktisolierung verbleiben 110 g eines klaren, farblosen Aziridinosilikons.

Synthesebeispiel 3:

Darstellung eines Aziridinosilikons aus einem Silikonöl mit seitenständigen Acrylatgruppen

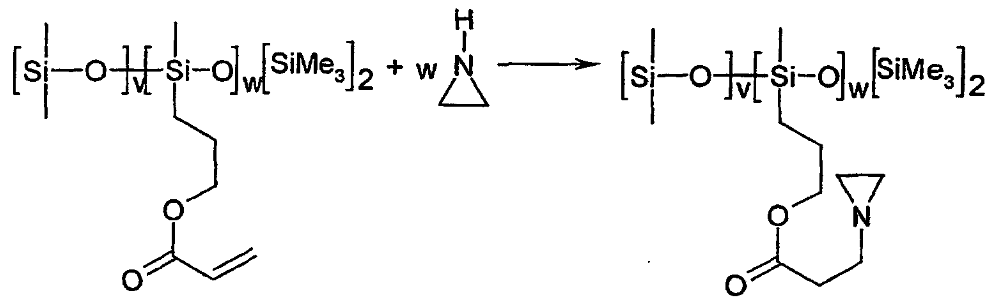

100 g eines Silikonöls UMS-182 (Gelest) [w = 0,18 * (v + w), 0,209 Mol Acrylatgruppen] werden mit 50 ml Toluol versetzt. Bei 30° C tropft man 18,01 g Aziridin [M = 43,07; 0,418 Mol] zu. Die Mischung wird auf 50° C erhitzt, nach fünf Stunden wird auf 100° C erhöht und solange bei 100° C gehalten, bis die Umsatzkontrolle mittels IR-Spektroskopie das Verschwinden der C=C-Doppelbindung anzeigt. Nach üblicher Aufarbeitung und Produktisolierung verbleiben 105 g eines klaren, farblosen Aziridinosilikons.

Herstellungsbeispiele Dentalmassen

Mit Hilfe von Laborknetem wurden die beschriebenen Katalysatorkomponenten K1 bis K3 im 100 g-Maßstab hergestellt. Die Herstellung der Basiskomponenten, die in Tabelle 1 beschrieben sind, erfolgte im 500 g-Maßstab.

In Tabelle 2 sind die Mischungen zusammengestellt, die unter Verwendung der beschriebenen Katalysatorkomponenten und der in Tabelle 1 beschriebenen Basiskomponente im jeweils angegebenen Gewichtsverhältnis untersucht wurden.

Die Mischungen gemäß Tabelle 2 wurden durch Anspateln auf den Mischblock innerhalb von 30 Sekunden zubereitet und zur Bestimmung der in Tabelle 2 zusammengestellten Eigenschaften eingesetzt.

Herstellung der Katalysatorkomonenten

Katalysatorkomponente K1

In einem Laborkneter wurden 44 g Acetyltributylcitrat (gemäß Bestandteil (C)) vorgelegt und 20 g β-(S-Lauryl-S-ethylsulfonium)butyronitrilfluoroborat (s. US-41 67 618, gemäß Bestandteil (B)) eingelöst. In diese Mischung wurden 12 g Diatomeenerde (gemäß Bestandteil (D)) und 24 g pyrogener Kieselsäure (HDK H 2000, Fa. Wacker, gemäß Bestandteil (D)) eingearbeitet

Katalysatorkomponente K2

In einem Laborkneter wurden 61,1 g eines Poly(-ethylen, -propylen)glykols mit einer Molmasse von 3400 g / Mol vorgelegt und schrittweise 21 g einer hydrophobierten Fällungskieselsäure (Sipemat D 17, Fa. Degussa, gemäß Bestandteil (D)) zugegeben.

9,9 g p-Toluolsulfonsäure-Monohydrat (gemäß Bestandteil (B)) wurden in 5 g destilliertem Wasser gelöst und der pastenförmigen Mischung zugesetzt. Nach Homogenisierung erfolgte die Zugabe einer Paste bestehend aus 2 g Zinkoxid (gemäß Bestandteil (B)) und 1 g Poly(-ethylen, -propylen)glykol (gemäß Bestandteil (C)) mit einer Molmasse von 3400 g/Mol. Die Paste wurde nach der letzten Zugabe noch eine Stunde geknetet.

Katalysatorkomponente K3

In einem Laborkneter wurden 19 g hydrophobierte Fällungskieselsäure (Sipernat D 17, Fa. Degussa, gemäß Bestandteil (D)) in 31 g eines Polypropylenoxid-diol (gemäß Bestandteil (C)) mit einer Molmasse von 2000 g / Mol

eingearbeitet. Dieser pastenförmigen Mischung wurde die Lösung einer Komplexverbindung (gemäß Bestandteil (B), hergestellt aus 3,6 g Borsäure und 17 g Salicylalkohol, in 29,4 g Polypropylenoxiddiol (gemäß Bestandteil (B)) zugegeben und die Paste eine Stunde geknetet.

Tabelle 1

| Zusammensetzung der Basiskomponenten | | | | | | |
|---|---|---|---|---|---|---|
| Verbindung | Gemäß Bestandteil | B1 | B2 | B3 | B4 | B5 |
| | | Gew.-% | | | | |
| Aziridinosilikon gemäß Synthesebeispiel 1 | (A) | 59,1 | 27,2 | 49,7 | - | 21,7 |
| Aziridinosilikon gemäß Synthesebeispiel 2 | (A) | - | 33,7 | - | 31,7 | 14,7 |
| Aziridinosilikon gemäß Synthesebeispiel 3 | (A) | - | - | 11,2 | 20,9 | 20,1 |
| Dimethylsiloxan AK 50 mit einer Viskosität von 50 mPas | (C) | 3,9 | 6,8 | 3,10 | 5,70 | 5,50 |
| Dimethylsiloxan AK 5000 mit einer Viskosität von 5000 mPas | (C) | 6,3 | 9,9 | - | - | 6,0 |
| Quarzpulver (Silbond TST 600, Frechen) | (D) | - | - | 25,3 | 40,0 | 32,0 |
| Diatomeenerde | (D) | 30,7 | 21,7 | 10,7 | - | - |
| Silikon-Polyether-Blockpolymer (Silwet L 7280) | (C) | - | 0,7 | - | 1,7 | - |

Die erfindungsgemäßen Basiskomponenten gemäß Tabelle 1 zeichneten sich durch eine sehr gute Lagerbeständigkeit aus.

So ließen sich die bei 36° C über einen Zeitraum von 9 Monaten eingelagerten Proben noch einwandfrei mit den angegebenen Katalysatorkomponenten verarbeiten und ergaben mechanische Kennwerte des Elastomerfestkörpers, die um weniger als 15 % von den Werten abwichen, die zu Beginn der Einlagerung gemessen wurden.

Tabelle 2

| Erfindungsgemäße Elastomermassen unter Verwendung der Katalysatorkomponenten 1 bis 3 und von Basiskomponenten gemäß Tabelle 1 sowie ermittelte Eigenschaften | | | | | | |
|---|---|---|---|---|---|---|
| | Verwendungsbeispiele Nr. | | | | | |
| | 1 | 2 | 3 | 4 | 5 | 6 |
| Katalysatorkomponente (K) | K2 | K3 | K2 | K1 | K2 | K3 |
| Basiskomponente (B) | B1 | B2 | B3 | B4 | B5 | B1 |
| Mischungsverhältnis (nach Gewicht) (K) : (B) | 1 : 4,7 | 1 : 5,0 | 1 : 5,0 | 1 : 4,3 | 1 : 5,1 | 1 : 5,0 |
| Verarbeitungszeit bei 23°C [a)] [Minuten] | 1,7 | 1,0 | 1,5 | 2,1 | 1,7 | 1,2 |
| Aushärtungsende bei 23°C [b)] [Minuten] | 5,3 | 3,7 | 3,9 | 7,0 | 5,5 | 4,1 |
| Reißdehnung [%] [a)] | 72 | 112 | 79 | 62 | 51 | 100 |
| Reißfestigkeit [MPa] [a)] | 0,82 | 0,75 | 0,92 | 0,69 | 0,87 | 1,03 |
| Shore A-Härte nach 24 h gemäß DIN 53505 | 42 | 39 | 47 | 49 | 52 | 43 |

[a)] gemäß DIN / EN 4823

[b)] Als Aushärtungsende wird der Zeitpunkt definiert, bei dem ein elastischer Festkörper vorliegt, der keine Oberflächenklebrigkeit besitzt

Alle Mischungen der erfindungsgemäßen Verwendungsbeispiele 1 bis 6 (Tabelle 2) erfüllten die Anforderungen an eine elastische Abformmasse nach DIN / EN 2482 und führten zu Formkörpern, die nach einer Lagerzeit bei

Raumtemperatur von 24 Stunden eine Shore A-Härte (s. Tabelle 2) deutlich über 20 besaßen.

**Patentansprüche**

**1.** N-Alkylaziridinosilikone der Formel:

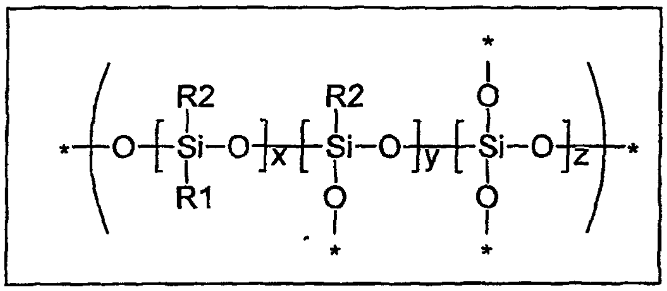

(1.1)

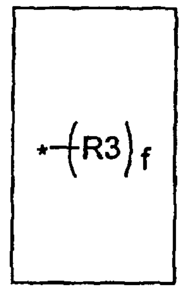

(1.2)

(1)

wobei:

R1 = H, $C_1$-$C_{12}$-Alkyl, $C_2$-$C_{12}$-Alkenyl, $C_2$-$C_{12}$-Alkinyl, $C_7$-$C_{15}$-Alkaryl, $C_7$-$C_{15}$-Aratkyl, $C_3$-$C_{12}$-Cycloalkyl bedeutet und diese Reste teilweise, ganz oder gemischt mit Cl oder F substituiert sein können und/oder 0 bis 5 Heteroatome aus der Gruppe O, N, S enthalten können,
R2 = ein Rest aus der Auswahl R1 und/oder R4 bedeutet, und
R3 = $SiR1_3$ oder $SiR1_2R4$,
mit R4 =

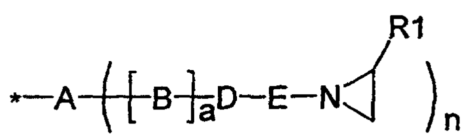

(2)

und

A = ein (n+1)-fach radikalischer gesättigter, ungesättigter oder aromatischer, linearer, verzweigter oder cyclischer Kohlenwasserstoffrest, der 0 bis 5 Heteroatome aus der Gruppe O, N, S enthalten kann und 1 bis 18 Kohlenstoffatome umfasst,
B = ausgewählt aus der Gruppe O, S, NR1,
D = ausgewählt aus der Gruppe C(O)O, C(O)NR1, C(O), C(O)C(O), $C(U)(CH_2)_m(C(O)$, C(S)NR1, $CH_2$,
E = ein 2-fach radikalischer gesättigter oder ungesättigter, linearer, verzweigter oder cyclischer Kohlenwasserstoffrest, der 0 bis 5 Heteroatome aus der Gruppe O, N, S enthalten kann und 0 bis 18 Kohlenstoffatome umfasst,
a = 0 oder 1,
f = eine ganze Zahl von 2 bis 1000,
n, m = eine ganze Zahl von 1 bis 10,

sowie

x, y, z jeweils entweder 0 oder ganze Zahlen darstellen, deren Summe zwischen 1 und 10.000 liegen soll, mit den Einschränkungen, dass, wenn x größer 0 ist, y oder z kleiner oder gleich x sind, bevorzugt kleiner oder gleich 0,05 mal x und besonders bevorzugt 0,02 mal x sein soll.

**2.** Härtbare Massen auf der Basis von N-Alkylaziridinosilikonen gemäß Anspruch 1, wobei diese nach der Mischung der Komponenten, jeweils bezogen auf 100 Masseteile, enthalten:

(A) 30 bis 97 Masseteile von mindestens einem N-Alkylaziridinoblockcopolymer mit Molmassen im Bereich von 500 bis 50.000 g / Mol und Aziridinoäquivaientmassen im Bereich von 250 bis 25.000 g / Äquivalent,
(B) 1 bis 10 Masseteile von Startersubstanzen, die geeignet sind, die Aushärtung der N-Alkylaziridinoblockcopolymeren zu bewirken,
(C) 1 bis 35 Masseteile von organischen Verdünnungsmitteln,
(D) 1 bis 50 Masseteile von Modifikatoren.

**3.** Zweikomponentige Dentalmassen, enthaltend N-Alkylaziridinosilikone gemäß Anspruch 1, bestehend aus einer Basiskomponente und einer Katalysatorkomponente gemäß Anspruch 1, wobei die durch homogene Mischung der Katalysatorkomponente und der Basiskomponente erzeugte Zubereitung eine Verarbeitungszeit bei Raumtemperatur zwischen 0,5 bis 10 Minuten besitzt und die Zubereitung in einem Temperaturbereich von 23 bis 36° C innerhalb einer Zeitspanne von einer bis 20 Minuten zu einer elastisch deformierbaren Masse mit einer Shore A-Härte von mindestens 20 aushärtet.

**4.** Massen nach einem der Ansprüche 2 oder 3, wobei sie als Bestandteil (A) N-Alkylaziridinosilikone mit mindestens einer und bis zu 10 N-Alkytaziridinogruppen pro Molekül enthalten und wobei die N-Alkylaziridinosilikone Molmassen im Bereich von 500 bis 50.000 g/Mol aufweisen.

**5.** Massen nach einem der Ansprüche 2 bis 4, wobei diese N-Alkylaziridinosilikone enthalten, die stark unterschiedliche Aziridinoäquivalentmassen besitzen.

**6.** Massen gemäß einem der Ansprüche 2 bis 5, wobei diese als Bestandteil (C) Organopolysiloxane mit Polyalkylenoxidblöcken enthalten, die folgenden Formeln folgen:

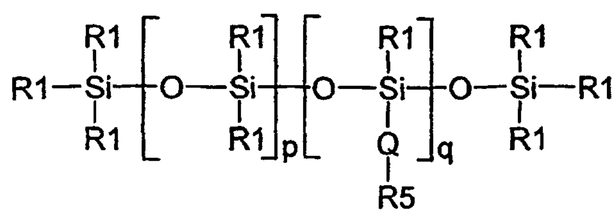

und/oder

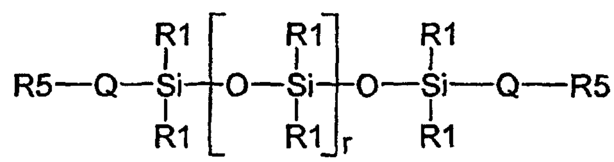

wobei bedeuten:

Q = eine α, ω- radikalische Polyetherkette der Formel (3):

$$-[O\text{-}C_gH_{2g}]_h- \qquad (3);$$

R5 = OH, OR1, O-C(O)-R1, O-C(O)-NHR1;
g = 2 bis 20;
h = 1 bis 1000;
p: eine ganze Zahl von 1 bis 1000;

q: eine ganze Zahl von 1 bis 50;
r: eine ganze Zahl von 1 bis 1000.

7. Zweikomponentige Dentalmasse nach einem der Ansprüche 2 bis 6, wobei als Starter Trisalkylsulfoniumsalze verwendet werden.

8. Zweikomponentige Dentalmasse nach einem der Ansprüche 2 bis 6, wobei als Starter Brönsted-Säuren verwendet werden, deren Acidität durch Antacida gemindert wurde.

9. Zweikomponentige Dentalmasse nach einem der Ansprüche 2 bis 6, wobei die Katalysatorkomponente mindestens einen Borsäurekomplex enthält.

10. Verwendung der N-Alkytaziridinosilikone gemäß Anspruch 1 für die Herstellung von härtbaren Massen.

11. Verwendung der N-Alkylaziridinosilikone gemäß Anspruch 1 für die Herstellung von Dentalmassen.

12. Verwendung der Massen nach einem der Ansprüche 2 bis 9 zum Verkleben, Beschichten oder Vergießen von Substraten sowie im Dentalbereich, insbesondere als dentale Abformmassen, Bissregistriermassen, Modellmaterialien, provisorische Verschlussmassen, Materialien zur Herstellung von provisorischen Kronen und Brücken sowie Dubliermassen.

13. Behältnis, enthaltend mindestens eine Masse nach mindestens einem der Ansprüche 2 bis 9.

## Claims

1. An N-alkylaziridinosilicone of the formula:

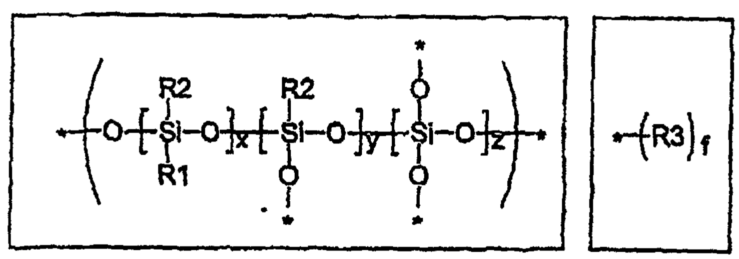

(1.1) (1.2)

(1)

where:

R1 = H, $C_1$-$C_{12}$-alkyl, $C_2$-$C_{12}$-alkenyl, $C_2$-$C_{12}$-alkynyl, $C_7$-$C_{15}$-alkaryl, $C_7$-$C_{15}$-aralkyl or $C_3$-$C_{12}$-cycloalkyl, and these radicals may be substituted partially, fully or in a mixed manner by Cl or F and/or may contain from 0 to 5 heteroatoms from the group consisting of O, N and S,
R2 = a radical from the selection of R1 and/or R4, and
R3 = $SiR1_3$ or $SiR1_2R4$,
where R4 =

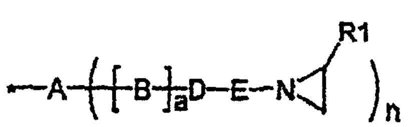

(2)

and

A = an (n+1)-valent saturated, unsaturated or aromatic, linear, branched or cyclic hydrocarbon radical, which may contain from 0 to 5 heteroatoms from the group consisting of O, N and S and comprises from 1 to 18 carbon atoms,
B = selected from the group consisting of O, S and NR1,
D = selected from the group consisting of C(O)O, C(O)NR1, C(O), C(O)C(O), C(O) $(CH_2)_m$(C (O), C(S)NR1 and $CH_2$,
E = a divalent saturated or unsaturated, linear, branched or cyclic hydrocarbon radical, which may contain from 0 to 5 heteroatoms from the group consisting of O, N and S and comprises from 0 to 18 carbon atoms,
a = 0 or 1,
f = an integer from 2 to 1000,
n and m = an integer from 1 to 10,

and

x, y and z are each either 0 or integers whose sum is between 1 and 10,000,

with the restrictions that, if x is greater than 0, y or z are less than or equal to x, preferably less than or equal to 0.05 times x and particularly preferably 0.02 times x.

**2.** A curable material based on N-alkylaziridinosilicones as claimed in claim 1, which, after mixing of the components, comprises, in each case based on 100 parts by weight:

(A) from 30 to 97 parts by weight of at least one N-alkylaziridino block copolymer having molecular weights in the range from 500 to 50,000 g/mol and aziridino equivalent weights in the range from 250 to 25,000 g/equivalent,
(B) from 1 to 10 parts by weight of starter substances which are suitable for effecting curing of the N-alkylaziridino block copolymers,
(C) from 1 to 35 parts by weight of organic diluents,
(D) from 1 to 50 parts by weight of modifiers.

**3.** A two-component dental material comprising N-alkylaziridinosilicones as claimed in claim 1, consisting of a base component and a catalyst component as claimed in claim 1, where the preparation produced by homogeneous mixing of the catalyst component and the base component has a pot life at room temperature of between 0.5 and 10 minutes, and the preparation cures within a period of from one to 20 minutes at a temperature in the range from 23 to 36°C to give an elastically deformable material having a Shore A hardness of at least 20.

**4.** A material as claimed in one of claims 2 or 3, which comprises, as constituent (A), N-alkylaziridinosilicones having at least one and up to 10 N-alkylaziridino groups per molecule, and where the N-alkylaziridinosilicones have molecular weights in the range from 500 to 50,000 g/mol.

**5.** A material as claimed in one of claims 2 to 4, which comprises N-alkylaziridinosilicones which have greatly different aziridino equivalent weights.

**6.** A material as claimed in one of claims 2 to 5, which comprise, as constituent (C), organopolysiloxanes containing polyalkylene oxide blocks which conform to the following formulae:

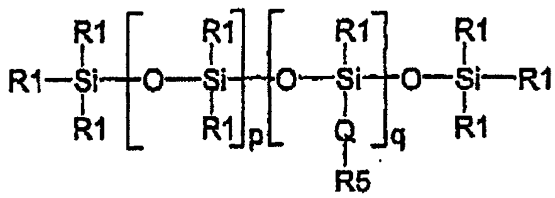

and/or

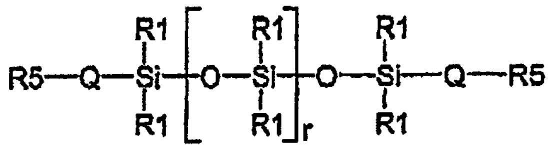

where:

Q = an $\alpha,\omega$-valent polyether chain of the formula (3) :

$$-[O-C_gH_{2g}]_h- \quad (3);$$

R5 = OH, OR1, O-C(O)-R1 or O-C(O)-NHR1;
g = from 2 to 20;
h = from 1 to 1000;
p = an integer from 1 to 1000;
q = an integer from 1 to 50;
r = an integer from 1 to 1000.

**7.** A two-component dental material as claimed in one of claims 2 to 6, where the starters used are trisalkylsulfonium salts.

**8.** A two-component dental material as claimed in one of claims 2 to 6, where the starters used are Brönsted acids whose acidity has been reduced by antiacids.

**9.** A two-component dental material as claimed in one of claims 2 to 6, where the catalyst component comprises at least one boric acid complex.

**10.** The use of N-alkylaziridinosilicones as claimed in claim 1 for the preparation of curable materials.

**11.** The use of N-alkylaziridinosilicones as claimed in claim 1 for the preparation of dental materials.

**12.** The use of materials as claimed in one of claims 2 to 9 for bonding, coating or encapsulation of substrates and in the dental sector, in particular as dental impression materials, bite registration materials, modeling materials, temporary filling materials, materials for the production of temporary crowns and bridges, and duplicating materials.

**13.** A container containing at least one material as claimed in at least one of claims 2 to 9.

## Revendications

**1.** N-alkylaziridinosilicones de formule :

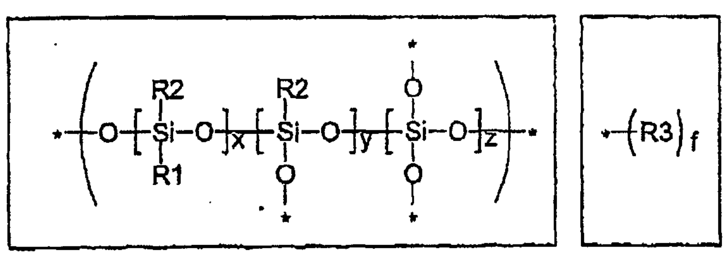

(1.1) (1.2)

(1)

dans laquelle

R1 représente H, un groupe alkyle en $C_1$-$C_{12}$, alcényle en $C_2$-$C_{12}$, alcynyle en $C_2$-$C_{12}$, alkaryle en $C_7$-$C_{15}$, aralkyle en $C_7$-$C_{15}$, cycloalkyle en $C_3$-$C_{12}$, et ces radicaux peuvent être substitués en partie, en totalité ou en mélange par Cl ou F et/ou peuvent contenir de 0 à 5 hétéroatomes choisis parmi O, N, S,

R2 représente un radical choisi parmi ceux de R1 et/ou R4, et

R3 représente $SiR1_3$ ou $SiR1_2R4$,
où R4 =

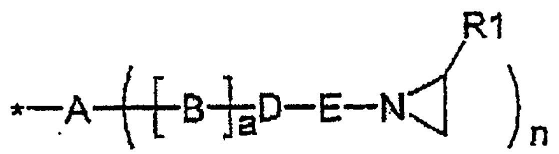

(2)

et

A représente un radical hydrocarboné saturé (n + 1) fois sur le radical, insaturé ou aromatique, linéaire, ramifié ou cyclique, qui peut contenir de 0 à 5 hétéroatomes choisis parmi O, N, S et comprend de 1 à 18 atomes de carbone,

B est choisi dans le groupe constitué par O, S, NR1,

D est choisi dans le groupe constitué par C(O)O, C(O)NR1, C(O), C(O)C(O), $C(O)(CH_2)_m$ C(O), C(S) NR1, $CH_2$,

E représente un radical hydrocarboné saturé deux fois dans le radical ou insaturé, linéaire, ramifié ou cyclique, qui peut contenir de 0 à 5 hétéroatomes choisis parmi O, N, S et comprend de 0 à 18 atomes de carbone,

a = 0 ou 1,

f est un nombre entier allant de 2 à 1 000,

n, m représentent un nombre entier allant de 1 à 10,

ainsi que

x, y, z représentent chacun soit 0, soit des nombres entiers dont la somme doit être comprise entre 1 et 10 000,

avec les limitations que lorsque x est supérieur à 0, y ou z est inférieur ou égal à x, de préférence doit être inférieur ou égal à 0,05 fois x, et de façon particulièrement préférée doit être égal à 0,02 fois x.

**2.** Matières durcissables à base de N-alkylaziridinosilicones selon la revendication 1, ces dernières, après le mélange des composants, contenant chacune, par rapport à 100 parties en masse :

(A) 30 à 97 parties en masse d'au moins un copolymère séquencé N-alkylaziridino ayant des masses moléculaires dans la plage de 500 à 50 000 g/mole et des masses équivalentes d'aziridino dans la plage allant de 250 à 25 000 g/équivalent,
(B) 1 à 10 parties en masse d'initiateurs appropriés à provoquer le durcissement des copolymères séquencés N-alkylaziridino,
(C) 1 à 35 parties en masse de diluants organiques,
(D) 1 à 50 parties en masse de modificateurs.

**3.** Matières dentaires à deux composants, contenant des N-alkylaziridinosilicones selon la revendication 1, constituées d'un composant de base et d'un composant catalyseur selon la revendication 1, la préparation produite par mélange homogène du composant catalyseur et du composant de base présentant un temps de mise en oeuvre à la température ambiante compris entre 0,5 à 10 minutes, et la préparation étant totalement durcie dans une plage de températures de 23 à 36°C en l'espace de 1 à 20 minutes, en une matière susceptible de déformation élastique, ayant une dureté Shore A d'au moins 20.

**4.** Matières selon la revendication 2 ou 3, contenant en tant que composant (A) des N-alkylaziridinosilicones comportant au moins 1 à 10 groupes N-alkylaziridino par molécule, et les N-alkylaziridinosilicones présentant des

masses moléculaires dans la plage allant de 500 à 50 000 g/mole.

5. Matières selon l'une quelconque des revendications 2 à 4, dans lesquelles ces matières contiennent des N-alkylaziridinosilicones qui présentent des masses d'équivalent aziridino très différentes.

6. Matières selon l'une quelconque des revendications 2 à 5, dans lesquelles ces matières contiennent en tant que composant (C) des organopolysiloxanes comportant des séquences polyoxyalkylène correspondant aux formules suivantes :

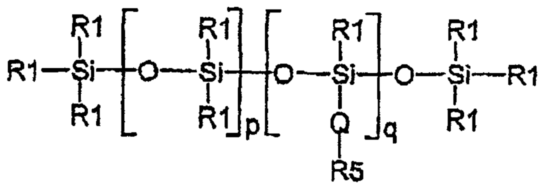

et/ou

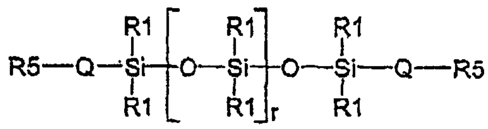

où

Q représente une chaîne polyéther $\alpha,\omega$-radicalaire de formule (3) :

$$-[O-C_gH_{2g}]_h- \qquad (3) ;$$

R5 = OH, OR1, O-C(O)-R1, O-C(O)-NHR1 ;
g = 2 à 20 ;
h = 1 à 1 000 ;
p représente un nombre entier allant de 1 à 1 000 ;
q représente un nombre entier allant de 1 à 50 ;
r représente un nombre entier allant de 1 à 1 000.

7. Matière dentaire à deux composants selon l'une quelconque des revendications 2 à 6, dans laquelle des sels de trialkylsulfonium sont utilisés en tant qu'amorceur.

8. Matière dentaire à deux composants selon l'une quelconque des revendications 2 à 6, dans laquelle on utilise comme amorceur des acides de Brönsted dont l'acidité a été diminuée par des antiacides.

9. Matière dentaire à deux composants selon l'une quelconque des revendications 2 à 6, dans laquelle le composant catalyseur contient au moins un complexe d'acide borique.

10. Utilisation des N-alkylaziridinosilicones selon la revendication 1, pour la préparation de matières durcissables.

11. Utilisation des N-alkylaziridinosilicones selon la revendication 1, pour la préparation de matières dentaires.

12. Utilisation des matières selon l'une quelconque des revendications 2 à 9, pour le collage, le revêtement ou la coulée de supports ainsi que dans le secteur dentaire, en particulier en tant que matières de moulage dentaires, matières de bis-enregistrement, matériaux modèles, matières d'obturation provisoire, matériaux pour la fabrication

de bridges et couronnes provisoires, ainsi que matières de placage.

**13.** Récipient contenant au moins une matière selon au moins l'une quelconque des revendications 2 à 9.